# EUROPEAN PATENT SPECIFICATION

(11) **EP 2 194 916 B1**
(45) Date of publication and mention of the grant of the patent: **11.11.2015**
(21) Application number: 08807681.5
(22) Date of filing: 16.09.2008
(51) Int. Cl.: A61F 2/04, A61F 2/02, B29C 47/02

(54) **A TUBULAR WORKPIECE FOR PRODUCING AN IMPROVED BALLOON CUFF TRACHEOSTOMY TUBE, AND METHOD**
ROHRFÖRMIGES WERKSTÜCK ZUR HERSTELLUNG EINES VERBESSERTEN TRACHEOSTOMIETUBUS MIT BALLONMANSCHETTE, UND VERFAHREN
PIÈCE TUBULAIRE POUR PRODUIRE UN TUBE DE TRACHÉOTOMIE À BALLONNET AMÉLIORÉ, ET PROCÉDÉ

(30) Priority: 20.09.2007 US 994664 P; 08.09.2008 US 206480
(43) Date of publication of application: 16.06.2010
(73) Proprietor: Avent, Inc., Alpharetta, GA 30004 (US)
(72) Inventor: CUEVAS, Brian, J., Cumming Georgia 30040 (US); SCHUMACHER, James, F., Cumming GA 30041 (US); KENOWSKI, Michael, A., Alpharetta GA 30005 (US); TEIXEIRA, Scott, M., Cumming GA 30040 (US)
(74) Representative: Davies, Christopher Robert
(86) International application number: PCT/IB2008/053755
(87) International publication number: WO 2009/037640

(56) References cited:
- WO-A1-97/04829
- WO-A2-01/85229
- US-A- 4 886 059
- US-A- 5 653 230
- US-A1- 2005 166 926
- US-B1- 6 286 509
- US-B1- 6 526 977
- US-B2- 6 612 305
- DATABASE WPI Week 200369 Thomson Scientific, London, GB; AN 2003-726601 XP002716532, -& JP 2003 275319 A (TERUMO CORP) 30 September 2003 (2003-09-30)

## Description

### BACKGROUND

Balloons or "cuffs" for tracheostomy (trach) tubes are sized to obdurate the trachea of a patient after they are inflated so that breathing takes place only through the lumen of the trach tube. The trach tube is designed to be inserted into the trachea from the front of the throat after it has been punctured and dilated to receive the tube. The balloon is located near the distal end of the tube and is typically inflated using a small conduit or inflation lumen after the tube has been inserted into the tracheal stoma and is in place in the trachea. The proximal end of the tube may be connected to a mechanical ventilator or respirator if necessary to provide assisted breathing.

Trach tube balloons are desirably soft and compliant so that damage to the tracheal tissue is kept to a minimum. Prior trach tube balloons that operated at relatively high pressures and had relatively thick walls caused considerable damage to the trachea. Newer designs having much thinner walls and that operate at lower pressures have reduced the trauma considerably. One example of such a balloon is that taught by Gobel in US patent 6,526,977 which provides a thin (at most 20 micron) balloon wall and which is effective at low pressures; 30 mbar or less.

Recent work by Fauza (US patent 6,612,305) provides a shaped balloon that is elongated so that it adheres to a larger area of the internal lining of the trachea to provide for better control over the location of the balloon, i.e. better "anchorage". This balloon appears to completely block the tracheal stoma and so inhibit the ability to drain any secretions that may pool above the balloon.

The draining of secretions from above the balloon is important because they are a prime suspect in the cause of ventilator assisted pneumonia (VAP). VAP is a serious and sometimes fatal problem that may occur in patients intubated for long periods of time. While VAP is generally less common in patients having trach tubes as contrasted with endotracheal tubes, it is still a serious concern.

While the previous balloons designed by Gobel and Fauza perform well, it would be quite desirable to have the ability to control the wall thickness of the balloon at various points or areas on the balloon. It may be desired for certain applications, for example to produce a balloon that is thicker or thinner at the front or rear of the trachea. Current balloon making technology uses generally symmetrical polymeric tubing that is blown into a balloon shape. This process results in a balloon having generally symmetrical wall thicknesses or results in predictable (but uncontrollable) thickness changes based on the degree of blowing of each wall section.

It would be desirable to give the balloon designer another dimension of control in order to tailor the balloon design more precisely or to allow for creative development of even more improved balloons.

JP 2003 275319A discloses an asymmetric balloon catheter for selectively expanding a blood vessel wall.

### SUMMARY

The present invention provides an inflatable balloon as claimed in claim 1. The present invention also provides a method of blow-molding a tubular workpiece into an inflatable balloon as claimed in claim 9.

This disclosure concerns a tubular workpiece having a lumen that may be used to make balloons for use on a tracheostomy tube. The workpiece is asymmetrical, allowing the balloon designer to more precisely control the thickness of the walls of the balloon in different areas.

The tubular workpiece may further include a lengthwise marking or line that is visible to a user, to help show the orientation of the workpiece in the mold.

The balloon may be made by placing the workpiece in a mold, heating the mold to soften the workpiece and pressurizing the tube internally (i.e. blowing) to force it to expand and conform to the walls of the mold. Rotating the asymmetrical tubular workpiece within the mold prior to blowing the balloon can change the location of thinner and thicker areas of the walls of the formed balloon.

The tracheostomy tube device includes a conventional hollow tube having a proximal end portion, a distal end portion, and a bend region intermediate of the end portions. The distal end portion of the tube is arranged for insertion through a patient's throat and tracheal stoma and into the tracheal lumen such that the distal end portion of the tube extends in a first direction within the tracheal lumen when the proximal end portion extends in a second direction through the tracheal stoma. The balloon that may be produced from the tubular workpiece may be attached to the hollow tube by conventional means. The balloon has a distal balloon portion substantially centered about and attached to the distal end portion of the tube. The balloon also has a proximal balloon portion attached to the bend region of the tube and positioned substantially off-center about the bend region below the proximal plane of the device. Upon inflation, this configuration provides for expansion of the balloon around the distal end portion of the tube and the proximal end portion of the tube below the proximal plane of the device to seal the trachea below the tracheal stoma and avoid sealing the trachea above the tracheal stoma. Desirably, this configuration of the balloon will allow secretions to exit the stoma.

The device further includes means for inflating and deflating the balloon. These means for inflating and deflating the balloon may be conventional flexible tubing placed along the trach tube and through the flange of the trach tube.

### BRIEF DESCRIPTION OF THE DRAWINGS

Figures 1A and Figure 1B are each an illustration of cross-section of an exemplary asymmetric raw tube having a lumen.
Figure 2 is a perspective view of an inflatable balloon component.
Figure 3 is an illustration of an exemplary device in which the balloon is inflated to seal the trachea in the region below the tracheal stoma while avoiding sealing the trachea in the region above the tracheal stoma.

### DETAILED DESCRIPTION

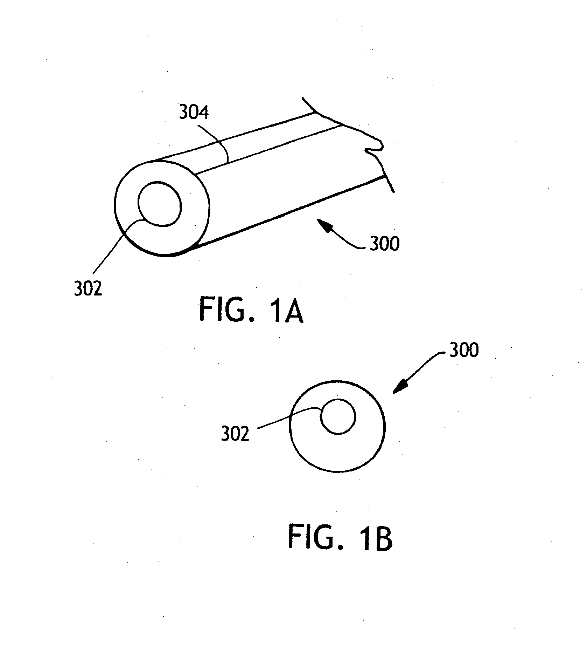

The asymmetrical tubular workpiece described generally above is illustrated in Figures 1A and 1B wherein the raw tube 300 has a lumen 302. The lumen is asymmetrical, meaning that the lumen is not exactly centered in the tube but is off center, resulting in varying wall thickness. The tube 300 may further have a line 304 that is located where the lumen is closest to the outside wall of the tube. The optional line 304 may be located at other locations, of course, depending on the needs of the user. The line 304 may be used to align the tube in a mold as desired.

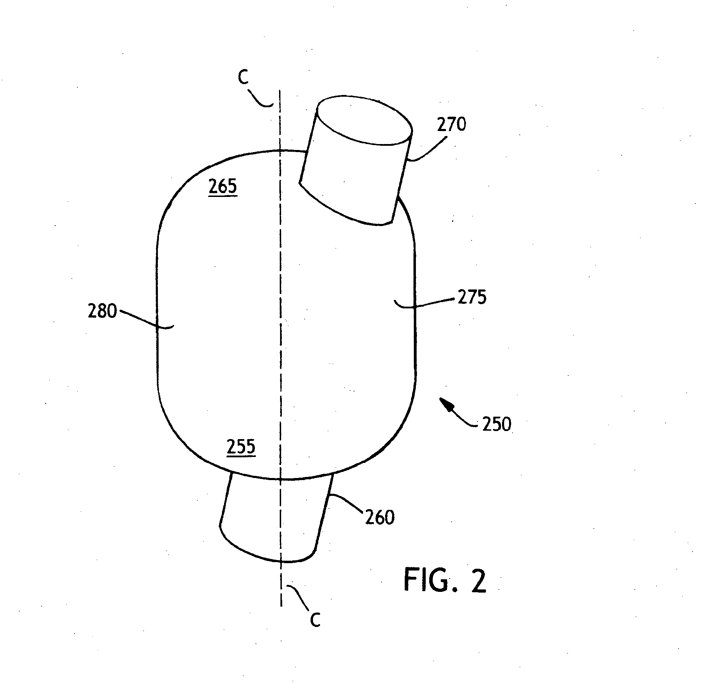

The tube may be placed in a mold for forming into a balloon or "blow molding". Figure 2 shows and example of a balloon that may be formed (blown) using the asymmetrical tube. Figure 2 is, of course, necessarily a representation of the interior of the mold as well since the tube expands to contact the entire interior of the mold. The raw tube may be placed in the mold so that it lies in a straight line and so that either end protrudes from the mold. The mold and tube are heated by conventional means, pressure is applied to the tube internally (in the lumen), and the tube expands to fill the mold, forming the balloon. After the balloon is formed, the mold and balloon are cooled and the balloon is withdrawn from the mold. The balloon may be attached to a tracheostomy tube by means known in the art.

As should be clear from Figure 2, the balloon 250 has a distal end 255, a distal attachment zone 260, a proximal end 265, a proximal attachment zone 270 and walls on either side 275, 280 joining the distal end and the proximal end of the balloon, the walls being asymmetrical or not symmetrical. The asymmetrical design of the mold in this case results in the tubular workpiece (tube) being offset from the centerline (i.e., a central line or plane "C" along the longest dimension of the balloon or mold dividing the balloon or mold into two equal volumes), resulting in expansion of the tube that is not uniform. Generally speaking, the central line "C" is equidistant from the side 275 and the side 280. If the mold or balloon has dimensions that were generally the same from a front region to a back region and from side to side or if the mold or balloon were generally spherical, the central line "C" would generally be a line that divided the mold or balloon into two equal volumes. An asymmetrical mold is one in which one or both openings in the ends of the balloon produced from it are not centered on the ends or on the centerline. In an asymmetrical mold, the side of the tube that forms the larger side 280 of the balloon expands more than the side of the tube that forms the smaller side 275 of the balloon. A symmetrical raw tubing that is used to form a balloon in this manner will result in a balloon having differential wall thickness with the larger side 280 being much thinner than the smaller side 275.

The overall dimensions of balloons produced using the asymmetrical workpiece disclosed herein for tracheostomy tubes generally, from the upper region 275 to the lower region 280, may range from about 50 millimeters to about 25 millimeters and may desirably be between about 35 millimeters to about 30 millimeters. The dimensions from the distal end 405 to the proximal end 415 may range from about 60 millimeters or more to about 25 millimeters and may desirably be between about 40 millimeters to about 30 millimeters. Of course, it is contemplated that the dimensions may be larger or smaller.

If an asymmetrical tube is placed in an asymmetrical mold with the thicker part of the tube aligned to face the large side of the to-be-formed balloon, the balloon so formed will have wall thicknesses that are more uniform than those formed from a symmetrical tube. Should it be desired to produce an asymmetrical balloon having walls 275, 280 that are approximately the same in thickness, an arrangement not in accordance with the present invention, the asymmetrical tube disclosed herein should be used.

In accordance with the present invention, the asymmetrical tube is aligned in the mold so that the thinner part of the tube is facing the large side. This will result in a differential thickness of the balloon walls being increased beyond that formed by the symmetrical tube.

A symmetrical mold may also be used with the asymmetrical tube disclosed herein in an arrangement not in accordance with the present invention. A symmetrical mold is one producing a balloon having its openings approximately centered on either end and with an approximately cylindrical shape.

The balloon formed using asymmetrical tube will have differential wall thicknesses where one side of the balloon has thinner walls and the other side of the balloon has thicker walls even though the shape of the balloon so formed is symmetrical. Typical dimensions for such symmetrical balloons are from between 20 and 60 mm in length and 20 to 30 mm in diameter, though balloons for pediatric patients may be smaller. Wall thicknesses are desired to be less than 30 microns at the thickest location and more desirably less than 20 microns.

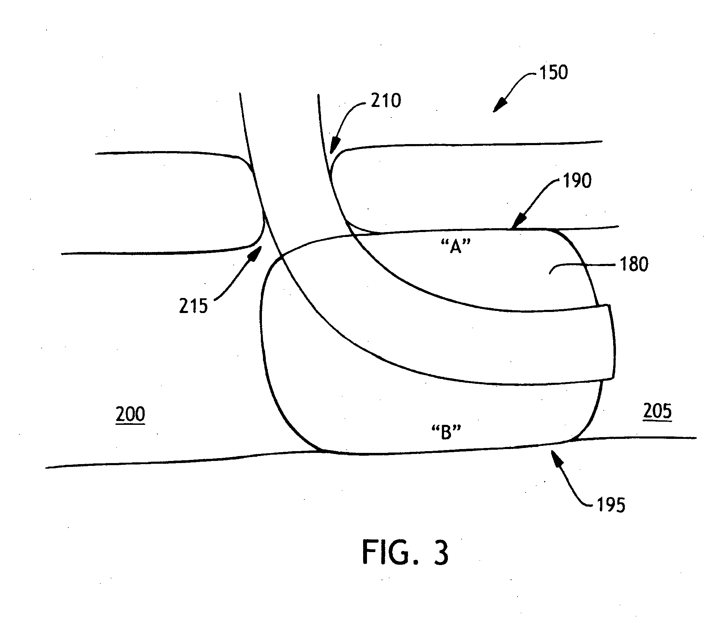

Turning to Figure 3, there is shown a cross-section of a tracheostomy tube device 150 in the trachea 200 of a patient. The tracheostomy tube device 150 may have balloon walls that are non-uniform thickness made using the asymmetrical tube disclosed herein. For example, the device may have a first portion "A" of the balloon in which the walls have a thickness of about 20 to 30 micrometers and a second portion "B" of the balloon in which the walls have a thickness of about 5 to about 15 micrometers.

The measurement of balloon wall thicknesses may be made using a Litematic device. An exemplary device is the series 318 Model VL-50A by Mitutoyo America Corporation. According to the manufacturer, the Litematic device measures thicknesses between 0 and 50.8 mm with a resolution of 0.01 micron, using a probe tip and an inflexible ceramic base. The measuring force used is 0.01 N (1 gram). The probe tip used for testing herein was a 3 mm diameter carbide ball contact point which was provided as the "standard" probe tip with the Litematic device.

Strips of single-ply foils or membranes may be used to determine the thickness of each sample. Balloon specimens (not attached to a trach tube) from each sample may be cut to prepare the strips: first the ends should be cut off to leave a uniform band of about 30 mm in width; then each band should be cut in the width direction to form a strip. Thickness measurements at 10 locations along the length of each strip should be made, the individual measurements of strips for each sample (with at least 6 strips measured) should be averaged together, and the respective standard deviations calculated.

It is desirable that the first portion "A" of the balloon 180 is the portion of the balloon contacting the upper portion of a cross-sectional region of the tracheal lumen 200 and the second portion "B" of the balloon is the portion of the balloon contacting the lower portion of the same cross-sectional region of the tracheal lumen. More specifically with reference to Figure 3, the inflated balloon 180 is adapted to seal the trachea (i.e., the tracheal lumen 200) in the region 205 below the tracheal stoma 210 and avoid sealing the trachea in the region above the tracheal stoma. Desirably, this configuration of the balloon allows secretions to exit the stoma at opening 215 and provides increased control over the location of the balloon in the trachea, i.e. better "anchorability".

Although the inventors should not be held to a particular theory of operation, it is generally thought that having the relatively thinner second portion "B" of the balloon contacting the lower wall 195 of the trachea will provide a better seal in that region where secretions may be more prone to collect due to gravity when a patient is resting horizontally on his or her back. The relatively thicker first portion "A" of the balloon contacting the upper wall 190 of the trachea where secretions may be less prone to collect due to gravity when a patient is resting horizontally on his back.

Desirable inflation pressures for tracheostomy tube balloons of the type disclosed herein is generally between 20 and 30 mbar.

According to the invention, the raw tubular workpiece may be formed from thermoplastic polyurethane polymers, thermoplastic polyolefin elastomers, thermoplastic polyolefin block copolymers, SBS di-block elastomers, SEBS triblock elastomers, polyvinyl chloride (PVC), polyethylene terephthalate (PET) and blends and mixtures thereof. More desirably, polyurethane may be used because it has been found to cause less irritation to tissues than other materials. Useful polyurethanes include those from the Dow Chemical Company (Dow Plastics) available under the tradename Pellethane®. Pellethane® thermoplastic polyurethane elastomer is available in a number of grades and hardnesses and the particular one selected for a specific use will depend on the properties desired in the final product. The hardness of a polymer, for example, is an attribute that may be varied to meet the requirements of various applications. One exemplary polyurethane is designated Pellethane® 2363-90A and has a durometer hardness of 90A (ASTM D-2240). This polyurethane has a softening temperature of 110° C (ASTM D-790) and a melt index of 30 g/10 min. at 224 °C, 2160 g (ASTM D-1238).

The asymmetrical tubular workpiece disclosed herein may have dimensions for the outside diameter (OD) between 6 and 11 mm. The wall thickness may be between 70 and 150 microns. One example of an asymmetrical tubular workpiece may have an OD of 8.6 mm and a wall thickness varying between 80 and 120 microns. Of course, other dimensions for the raw tubular workpiece are contemplated and may be varied as required depending on the size dimensions and wall thickness dimensions desired for the resulting balloon.

The present invention also encompasses an inflatable balloon that has been blow molded from the tubular workpiece described above. The inflatable balloon has a distal end, a proximal end, distal attachment zone, a proximal attachment zone, a first wall and a second wall joining the distal end and the proximal end, wherein the first wall and the second wall are asymmetrical.

## Claims

1. An inflatable balloon (250) for use in tracheostomy that has been blow molded in an asymmetric mold from a tubular workpiece, the tubular workpiece comprising a raw tube (300) having a lumen (302) and composed of a thermoplastic polymer, the tube (300) having an asymmetric wall thickness such that the tube may be blow molded in a mold to form a balloon component,
wherein the inflatable balloon (250) has a distal end (255), a proximal end (265), distal attachment zone (260), a proximal attachment zone (270), a first wall (275) and a second wall (280) joining the distal end (255) and the proximal end (265), **characterised in that** said asymmetrical workpiece is placed in the asymmetrical mold with the thicker part of the workpiece aligned to face a small side of the mold, and wherein the balloon (250) so formed will have wall thicknesses that are less uniform than those formed from a symmetrical tube.

2. The balloon (250) of claim 1 having a distance from the distal end (255) to the proximal end (265) of between about 25 millimeters to about 60 millimeters and may desirably be between about 35 millimeters to about 30 millimeters.

3. The balloon (250) of claim 1 wherein said balloon walls (275,280) have a thickness less than 30 microns at the thickest location and said balloon (250) is between 20 and 60 mm in length and 20 to 30 mm in diameter.

4. The balloon 12501 of claim 1 herein the tubular workpiece comorises thermoplastic polyurethane polymers, thermoplastic polyolefin elastomers, thermoplastic polyolefin block copolymers, SBS di-block elastomers, SEBS triblock elastomers, poly vinyl chloride, polyethylene terephthalate and blends and mixtures thereof.

5. The balloon (250) of claim 1 wherein the tubular workpiece has an outside diameter between 6 and 11 mm and a wall thickness between 70 and 150 microns.

6. The balloon (250) of claim 1 wherein the tubular workpiece further comprises a lengthwise line along an exterior surface.

7. The balloon (250) of claim 1 wherein the tubular workpiece has been blow-molded in a symmetrical mold into the inflatable balloon (250) wherein said balloon (250) has differential wall thicknesses, the balloon (250) having an upper region and a lower region, wherein the upper region (275) has a thickness of from about 15 to about 30 micrometers and the lower region (280) has a thickness of from about 5 to about 15 micrometers.

8. The balloon (250) of claim 1 wherein the tubular workpiece that has been blow-molded in an asymmetrical mold into the inflatable balloon wherein said balloon has differential wall thicknesses, the balloon (250) having an upper region (275) and a lower region (280), wherein the upper and lower region (275,280) have a thickness of from about 15 to about 30 microns.

9. A method of blow-molding a tubular workpiece into an inflatable balloon (250) in an asymmetric mold, the inflatable balloon (250) having a distal end (225), a proximal end (265), distal attachment zone (260), a proximal attachment zone (270), a first wall (275) and a second wall (280) joining the distal end (255) and the proximal end (265), the tubular workpiece comprising a raw tube having a lumen and composed of a thermoplastic polymer, the tube having an asymmetric wall thickness, the method **characterised in** comprising:
placing the asymmetrical workpiece in an asymmetrical mold with the thicker part of the workpiece aligned to face a small side of the mold; and
blow molding the workpiece in the mold to form the inflatable balloon,
wherein the balloon (250) is formed to have wall thicknesses that are less uniform than those formed from a symmetrical tube.

10. The method of claim 9, wherein said balloon walls have a thickness less than 30 microns at the thickest location and said balloon is between 20 and 60 mm in length and 20 to 30 mm in diameter.

## Patentansprüche

1. Ein aufblasbarer Ballon (250) zur Verwendung bei einer Tracheostomie, welcher in einer asymmetrischen Form aus einem röhrenförmigen Werkstück blasgeformt wurde, wobei das röhrenförmige Werkstück eine Rohkanüle (300) aufweist, die ein Lumen (302) hat und aus einem thermoplastischen Polymer besteht, wobei die Kanüle (300) eine asymmetrische Wanddicke hat, so dass die Kanüle in einer Form blasgeformt werden kann, um eine Ballonkomponente zu bilden,
wobei der aufblasbare Ballon (250) ein distales Ende (255), ein proximales Ende (265), eine distale Befestigungszone (260), eine proximale Befestigungszone (270), eine erste Wand (275) und eine zweite Wand (280), welche das distale Ende (255) und das proximale Ende (265) zusammenfügen, aufweist, **dadurch gekennzeichnet, dass** das besagte asymmetrische Werkstück in der asymmetrischen Form so platziert ist, dass der dickere Teil von dem Werkstück ausgerichtet ist, um einer kleinen Seite von der Form gegenüberzuliegen, und wobei der so gebildete Ballon (250) Wanddicken haben wird, welche weniger einheitlich sind als diejenigen, die aus einer symmetrischen Kanüle geformt sind.

2. Der Ballon (250) von Anspruch 1 mit einer Distanz von dem distalen Ende (255) zu dem proximalen Ende (265) von zwischen etwa 25 Millimeter bis etwa 60 Millimeter, welche wünschenswerterweise zwischen etwa 35 Millimeter bis etwa 30 Millimeter sein kann.

3. Der Ballon (250) von Anspruch 1, wobei die besagten Ballonwände (275, 280) an der dicksten Stelle eine Dicke geringer als 30 Mikrometer haben und der besagte Ballon (250) zwischen 20 und 60 mm in der Länge und 20 bis 30 mm im Durchmesser ist.

4. Der Ballon (250) von Anspruch 1, wobei das röhrenförmige Werkstück thermoplastische Polyurethanpolymere, thermoplastische Polyolefinelastomere, thermoplastische Polyolefinblockcopolymere, SBS-Di-Block-Elastomere, SEBS-Tri-Block-Elastomere, Polyvinylchlorid, Polyethylenterephthalat und Mischungen und Vermischungen davon aufweist.

5. Der Ballon (250) von Anspruch 1, wobei das röhrenförmige Werkstück einen äußeren Durchmesser zwischen 6 und 11 mm und eine Wanddicke zwischen 70 und 150 Mikrometer hat.

6. Der Ballon (250) von Anspruch 1, wobei das röhrenförmige Werkstück weiter eine Längslinie entlang einer äußeren Oberfläche aufweist.

7. Der Ballon (250) von Anspruch 1, wobei das röhrenförmige Werkstück in einer symmetrischen Form in den aufblasbaren Ballon (250) blasgeformt wurde, wobei der besagte Ballon (250) unterschiedliche Wanddicken hat, wobei der Ballon (250) einen oberen Bereich und einen unteren Bereich hat, wobei der obere Bereich (275) eine Dicke von etwa 15 bis etwa 30 Mikrometer hat und der untere Bereich (280) eine Dicke von etwa 5 bis etwa 15 Mikrometer hat.

8. Der Ballon (250) von Anspruch 1, wobei das röhrenförmige Werkstück, welches in einer asymmetrischen Form in den aufblasbaren Ballon blasgeformt wurde, wobei der besagte Ballon unterschiedliche Wanddicken hat, wobei der Ballon (250) einen oberen Bereich (275) und einen unteren Bereich (280) hat, wobei der obere und untere Bereich (275, 280) eine Dicke von etwa 15 bis etwa 30 Mikrometer haben.

9. Ein Verfahren zum Blasformen eines röhrenförmigen Werkstücks in einen aufblasbaren Ballon (250) in einer asymmetrischen Form, wobei der aufblasbare Ballon (250) ein distales Ende (255), ein proximales Ende (265), eine distale Befestigungszone (260), eine proximale Befestigungszone (270), eine erste Wand (275) und eine zweite Wand (280), welche das distale Ende (255) und das proximale Ende (265) zusammenfügen, aufweist, wobei das röhrenförmige Werkstück eine Rohkanüle aufweist, die ein Lumen hat und aus einem thermoplastischen Polymer besteht, wobei die Kanüle eine asymmetrische Wanddicke hat, wobei das Verfahren **dadurch gekennzeichnet ist, dass** es aufweist:
Platzieren des asymmetrischen Werkstücks in einer asymmetrischen Form, sodass der dickere Teil von dem Werkstück ausgerichtet ist, um einer kleinen Seite von der Form gegenüberzuliegen; und
Blasformen des Werkstücks in der Form, um den aufblasbaren Ballon zu bilden, wobei der Ballon (250) gebildet wird, um Wanddicken zu haben, welche weniger einheitlich sind als diejenigen, die aus einer symmetrischen Kanüle geformt sind.

10. Das Verfahren von Anspruch 9, wobei die besagten Ballonwände an der dicksten Stelle eine Dicke geringer als 30 Mikrometer haben und der besagte Ballon zwischen 20 und 60 mm in der Länge und 20 bis 30 mm im Durchmesser ist.

## Revendications

1. Ballonnet gonflable (250) destiné à être utilisé pour une trachéotomie, qui a été moulé par soufflage dans un moule asymétrique à partir d'une pièce tubulaire, la pièce tubulaire comprenant un tube brut (300) ayant une lumière (302) et composé d'un polymère thermoplastique, le tube (300) ayant une épaisseur de paroi asymétrique de sorte que le tube peut être moulé par soufflage dans un moule afin de former un composant de ballonnet,
dans lequel le ballonnet gonflable (250) a une extrémité distale (255), une extrémité proximale (265), une zone de fixation distale (260), une zone de fixation proximale (270), une première paroi (275) et une seconde paroi (280) assemblant l'extrémité distale (255) et l'extrémité proximale (265), **caractérisé en ce que** ladite pièce asymétrique est placée dans le moule asymétrique avec la partie plus épaisse de la pièce alignée pour faire face à un petit côté du moule, et dans lequel le ballonnet (250) ainsi formé a des épaisseurs de paroi qui sont moins uniformes que celles formées à partir d'un tube symétrique.

2. Ballonnet (250) selon la revendication 1, ayant une distance à partir de l'extrémité distale (255) jusqu'à l'extrémité proximale (265) comprise entre environ 25 millimètres et environ 60 millimètres et peut, de manière souhaitable, être comprise entre environ 35 millimètres et environ 30 millimètres.

3. Ballonnet (250) selon la revendication 1, dans lequel lesdites parois de ballonnet (275, 280) ont une épaisseur inférieure à 30 microns au niveau de l'emplacement le plus épais et ledit ballonnet (250) est compris entre 20 et 60 mm de long et 20 à 30 mm de diamètre.

4. Ballonnet (250) selon la revendication 1, dans lequel la pièce tubulaire comprend des polymères thermoplastiques de polyuréthane, des élastomères thermoplastiques de polyoléfine, des copolymères blocs thermoplastiques de polyoléfine, des élastomères de SBS dibloc, des élastomères de SEBS tribloc, le polychlorure de vinyle, le polyéthylène téréphtalate et leurs mélanges.

5. Ballonnet (250) selon la revendication 1, dans lequel la pièce tubulaire a un diamètre externe compris entre 6 et 11 mm et une épaisseur de paroi comprise entre 70 et 150 microns.

6. Ballonnet (250) selon la revendication 1, dans lequel la pièce tubulaire comprend en outre une ligne dans le sens de la longueur le long d'une surface extérieure.

7. Ballonnet (250) selon la revendication 1, dans lequel la pièce tubulaire a été moulée par soufflage dans un moule symétrique en ballonnet gonflable (250), dans lequel ledit ballonnet (250) a des épaisseurs de paroi différentielles, le ballonnet (250) ayant une région supérieure et une région inférieure, dans lequel la région supérieure (275) a une épaisseur d'environ 15 à environ 30 micromètres et la région inférieure (280) a une épaisseur d'environ 5 à environ 15 micromètres.

8. Ballonnet (250) selon la revendication 1, dans lequel la pièce tubulaire a été moulée par soufflage dans un moule asymétrique en ballonnet gonflable, dans lequel ledit ballonnet a des épaisseurs de paroi différentielles, le ballonnet (250) ayant une région supérieure (275) et une région inférieure (280), dans lequel les régions supérieure et inférieure (275, 280) ont une épaisseur d'environ 15 à environ 30 microns.

9. Procédé pour mouler par soufflage une pièce tubulaire en un ballonnet gonflable (250) dans un moule asymétrique, le ballonnet gonflage (250) ayant une extrémité distale (225), une extrémité proximale (265), une zone de fixation distale (260), une zone de fixation proximale (270), une première paroi (275) et une seconde paroi (280) assemblant l'extrémité distale (255) et l'extrémité proximale (265), la pièce tubulaire comprenant un tube brut ayant une lumière et composé d'un polymère thermoplastique, le tube ayant une épaisseur de paroi asymétrique, le procédé étant **caractérisé en ce qu'**il comprend les étrapes consistant à :
placer la pièce asymétrique dans un moule asymétrique avec la partie plus épaisse de la pièce alignée pour faire face à un petit côté du moule ; et
mouler par soufflage la pièce dans le moule afin de former le ballonnet gonflable,
dans lequel le ballonnet (250) est formé pour avoir des épaisseurs de paroi qui sont moins uniformes que celles formées à partir d'un tube symétrique.

10. Procédé selon la revendication 9, dans lequel lesdites parois de ballonnet ont une épaisseur inférieure à 30 microns à l'emplacement le plus épais et ledit ballonnet est compris entre 20 et 60 mm de long et 20 à 30 mm de diamètre.
